# FASCICULE DE BREVET EUROPEEN

(11) **EP 0 613 668 B1**
(45) Date de publication et mention de la délivrance du brevet: **16.12.1998**
(21) Numéro de dépôt: 94420073.2
(22) Date de dépôt: 02.03.1994
(51) Int. Cl.: A61F 2/64, A61F 2/38

(54) **Prothèse cinematique externe du membre inférieur, notamment du genou**
Kinematische externe Prothese für eine untere Extremität, insbesondere ein Knie
Kinematic exernal prosthesis for a lower limb, notably a knee

(30) Priorité: 03.03.1993 FR 9302726
(43) Date de publication de la demande: 07.09.1994
(73) Titulaire: CENTRE STEPHANOIS DE RECHERCHES MECANIQUES HYDROMECANIQUE ET FROTTEMENT Société anonyme, F-42166 Andrezieux Boutheon Cédex (FR); Rigal, François, F-69450 Saint Cyr au Mont D'or (FR)
(72) Inventeur: Berger, Antoine, F-42230 Roche la Moliere (FR); Rigal, François, F-69450 Saint Cyr au Mont d'Or (FR)
(74) Mandataire: Thivillier, Patrick

(56) Documents cités:
- EP-A- 0 244 493
- DE-C- 489 216
- FR-A- 2 157 441
- FR-A- 2 470 588
- GB-A- 2 181 060

## Description

L'invention concerne plus particulièrement une articulation du genou pour une prothèse de membre après amputation de cuisse. Cette articulation pourra être couplée sur le plan fonctionnel à une articulation de cheville.

Le genou est l'articulation intermédiaire du membre inférieur qui concilie des impératifs de stabilité et de mobilité sous des contraintes en compression et cisaillements très importantes.

La mobilité du genou se déroule en un mouvement complexe associant un déplacement en flexion - rotation automatique.

La flexion du genou s'effectue dans le plan sagittal, selon une série d'axes transversaux dont la résultante de chacun d'eux constitue une spirale propre à chaque condyle et détermine la courbe des centres instantanés de rotation.

La rotation du genou s'effectue dans un plan horizontal selon une série d'axes verticaux dont la résultante de ceux-ci constitue une spirale associant celles décrites par la flexion du tibia sur les condyles et la rotation des condyles sur le tibia. Ce mouvement de rotation automatique couplée à la flexion - extension du genou se déroule de 0 à 90° en portant le tibia en rotation interne et en varus.

Il apparaît également que lors de la flexion, les condyles fémoraux roulent et glissent à la fois sur le plateau tibial. Pour le condyle interne, le roulement n'a lieu que pendant les 10 à 15 premiers degrés de flexion, tandis que pour le condyle externe, ce roulement se poursuit jusqu'à 20 degrés de flexion.

Lors de la rotation externe du tibia sous le fémur, le condyle externe avance dans la glène externe, tandis que le condyle interne recule dans la glène interne.

Lors de la rotation interne, le phénomène inverse se produit : le condyle externe recule dans sa glène tandis que l'interne avance dans la sienne.

En résumé, de manière importante, il apparaît que cette rotation automatique du genou, se traduit en fin de mouvement d'extension par une légère rotation externe, tandis que le début de la flexion comporte une rotation interne réduite, ces mouvements s'effectuant automatiquement, sans qu'intervienne un acte volontaire.

Dans le document FR-A-2 157 441, on a décrit un ensemble prothétique pour prothèse du genou comprenant un élément fémoral et un élément tibial présentant des formes complémentaires d'articulation mâle-femelle d'appui et de glissement. La solution proposée ne permet néanmoins pas d'obtenir, en combinaison avec un mouvement de flexion correspondant au début de la flexion du genou, un mouvement de rotation de l'élément tibial.

Le problème que se propose de résoudre l'invention est de reproduire au moyen d'un ensemble prothétique, ces différents mouvements anatomiques de l'articulation naturelle du genou. Comme indiqué, l'ensemble prothétique est plus particulièrement applicable à la conception d'une exo-prothèse sans pour cela exclure une application à une prothèse interne ou endo-prothèse.

Pour résoudre un tel problème, il a été conçu et mis au point un ensemble prothétique conforme à la revendication 1.

Ces formes mâle et femelle complémentaires se comportent comme un engrenage à une dent pendant la rotation, puis, à partir d'un certain angle de flexion, comme un arbre dans un palier à axe fixe.

Le décalage des centres des segments courbes des parties mâle et femelle correspond à l'angle de rotation désirée lors de la flexion.

En outre, les formes mâle et femelle complémentaires permettent d'avoir toujours trois points d'appui, quel que soit le degré de flexion, tout en ayant pour objectif de tenir compte de la rotation automatique et de la flexion qui résulte d'un roulement et d'un glissement.

La prothèse permet d'obtenir une rotation automatique au début de la flexion, puis une flexion classique autour d'un axe.

Dans ce but, les formes complémentaires sont établies d'un des deux côtés de chaque élément et notamment du côté latéral interne. A l'opposé des formes complémentaires, chaque élément présente des agencements d'articulation.

Les agencements d'articulation sont constitués par un axe transversal ou par des formes complémentaires de pivotement et/ou de glissement.

Pour résoudre le problème posé de combiner la flexion et la rotation, l'axe transversal est accouplé à une partie fixe de l'élément tibial et présente, à son extrémité libre, une rotule engagée dans une forme complémentaire de l'élément fémoral.

Avantageusement, la forme mâle est établie au niveau de l'élément fémoral, tandis que la forme femelle est établie au niveau de l'élément tibial. La forme femelle est établie dans un organe rapporté semi-solidaire, avec capacité de mobilité angulaire limité dans le plan horizontal, pour assurer la fonction des ménisques.

Dans le cas d'une prothèse pour amputés, les éléments fémoraux et tibiaux sont asservis à un dispositif de mise en tension et d'amortissement qui peut être relié à un système actif de contrôle des mouvements de flexion - extension de la cheville.

L'invention est exposée, ci-après plus en détail à l'aide des dessins annexés, dans lesquels :
La figure 1 est une vue en perspective à caractère schématique, avant assemblage des principaux éléments constitutifs de l'ensemble prothétique selon l'invention.
La figure 2 est une vue de côté de l'ensemble en position d'extension.
La figure 3 est une vue correspondante à la figure 2 en position de flexion.
La figure 4 est une vue en plan correspondant à la figure 2.
La figure 5 est une vue en plan correspondant à la figure 3.
La figure 6 est une vue en coupe longitudinale de l'ensemble.
La figure 7 est une vue à grande échelle montrant les formes de la partie mâle de l'élément fémoral.
La figure 8 est une vue à grande échelle montrant les formes de la partie femelle de l'élément tibial.
La figure 9 est une vue à caractère schématique en perspective d'un exemple de réalisation d'une endo-prothèse.
La figure 10 est un exemple de montage d'une endo-prothèse.
La figure 11 est une vue de face d'une autre forme de réalisation de la prothèse.
La figure 12 est une vue en coupe transversale considérée selon la ligue 12.12 de la figure 11.

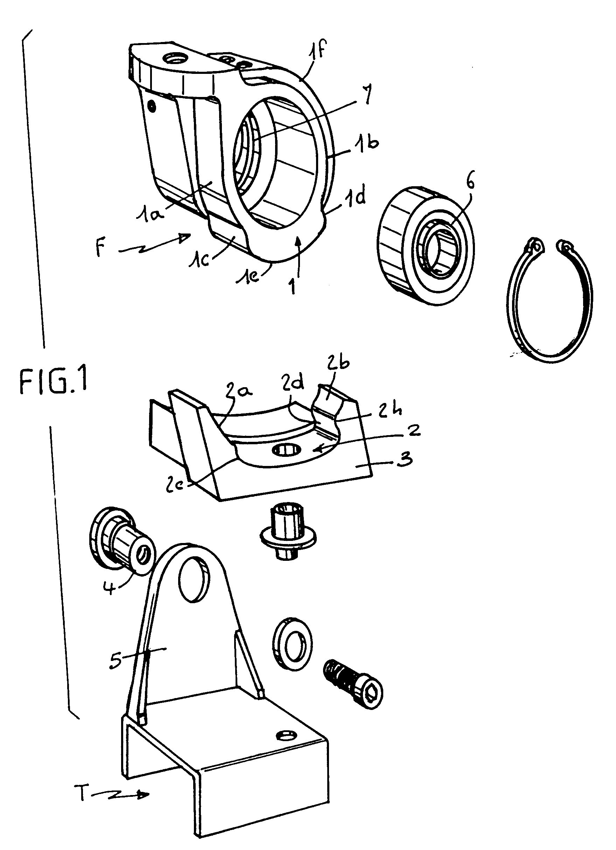

Comme le montre la figure 1, l'ensemble prothétique comprend un élément fémoral (F) et un élément tibial (T).

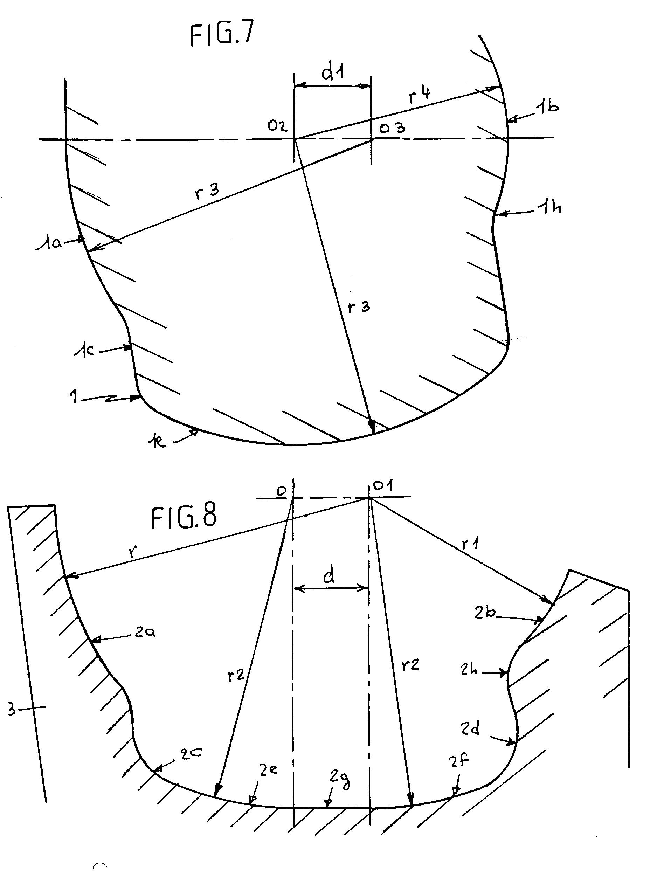

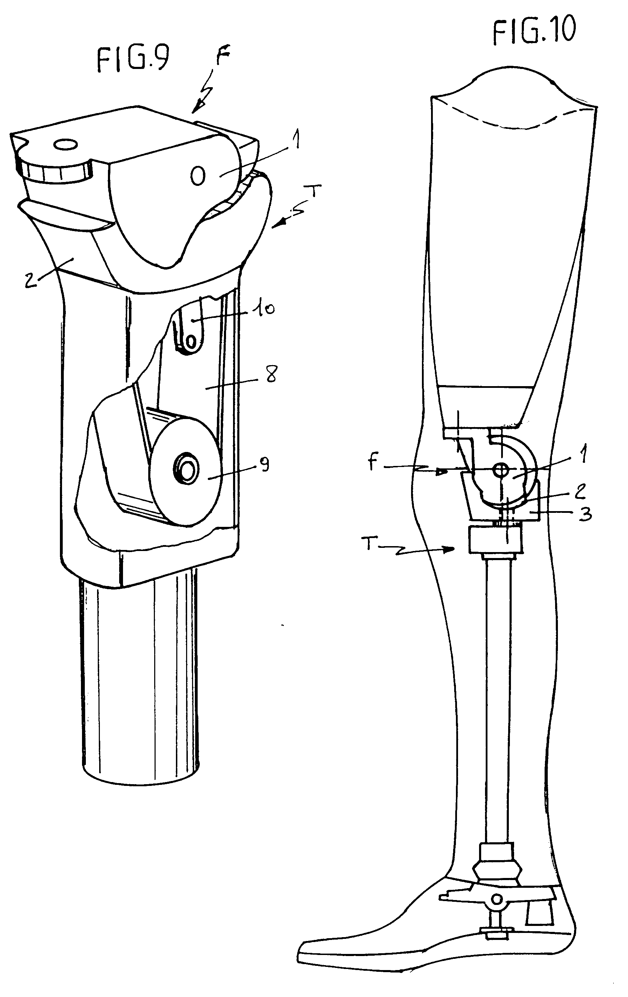

Selon une caractéristique à la base de l'invention, les éléments fémoral (F) et tibial (T) présentent des formes complémentaires d'articulation mâle (1) - femelle (2) d'appui et de glissement. Ces formes (1) et (2) présentent chacune des profils en section aptes à provoquer automatiquement, en combinaison avec un mouvement de flexion, un mouvement de rotation interne de l'élément tibial (T) par rapport à l'élément fémoral (F) selon un angle limité déterminé. Ces formes et profils complémentaires sont illustrés aux figures 7 et 8.

La forme femelle (2) (figure 8) est constituée par deux segments courbes concentriques (2a) et (2b). Ces deux segments (2a) et (2b) ont respectivement des rayons (r) et (r1) de longueurs différentes.

En outre, les segments (2a) et (2b) sont reliés par approximativement un quart de cercle (2c) (2d), respectivement à deux autres segments courbes (2e) et (2f). Les segments courbes (2e) et (2f) ont des rayons (r2) de longueurs identiques et égales à la longueur du rayon (r) du segment courbe (2a). Les centres (O) et (O1) des segments courbes (2e) et (2f) sont décalés et alignés d'une distance (d). Le centre (O1) du segment courbe (2f) correspond au centre du segment courbe (2b) de rayon (r1). Les deux segments courbes de raccordement (2e) et (2f) sont reliés par un segment rectiligne (2g), correspondant au décalage des centres (O) et (O1).

La forme mâle (1), figure 7, est constituée par deux segments courbes (1a) et (1b) de rayons différents (r3) et (r4). La longueur du rayon (r3) du segment courbe (1a) est égale à la longueur des rayons (r) et (r2). La longueur du rayon (r4) du segment courbe (1b) est égale à la longueur du rayon (r1) du segment courbe (2b) de la partie femelle (2). Les centres (O2) et (O3), des segments courbes (1a) et (1b), sont alignés et décalés d'une valeur (d1) correspondant au décalage (d) des centres (O) et (O1). Les segments courbes (1a) et (1b) sont prolongés angulairement par un segment rectiligne (1c) (1d) pour être reliés par un segment courbe (1e). La longueur du rayon (r3) du segment courbe (1e) est égale à la longueur des rayons (r) (r2)(r3).

Les segments courbe (1b) et rectiligne (1d) de la partie mâle (1) sont réunis par un rayon (1h), tandis que les segments courbes (2b) et (2d) de la partie femelle (2) sont réunis par un rayon (2h). La longueur du rayon (2h) est égale ou inférieure à la longueur du rayon (1h).

Les segments (1c) et (1d) sont parallèles et de longueurs différentes, le segment (1d) étant de longueur supérieure.

Les distances (d) et (d1) correspondent à l'angle de rotation désiré au début de la flexion. Il suffit donc d'augmenter ou de diminuer cette distance pour faire varier en conséquence, cette valeur angulaire.

Dans la forme de réalisation illustrée, les formes complémentaires (1) et (2) sont établies d'un des deux côtés de chaque élément (F) et (T), notamment du côté latéral interne. La forme mâle (1) est établie au niveau de l'élément fémoral (F) tandis que la forme femelle (2) est établie au niveau de l'élément tibial (T). Bien évidemment, on n'exclut pas une conception inverse.

Plus particulièrement, la forme femelle (2) est formée dans un organe rapporté (3) monté avec capacité de pivotement angulaire sur une partie fixe (5) de l'élément tibial (T). A l'opposé des formes complémentaires d'appui et de glissement (1) et (2), les éléments (T) et (F) sont accouplés par un axe transversal d'articulation (4). Cet axe (4) est monté dans la partie fixe (5) de l'élément tibial (T) et présente, à son extrémité libre, une rotule (6) engagée dans une empreinte complémentaire (7) de l'élément fémoral.

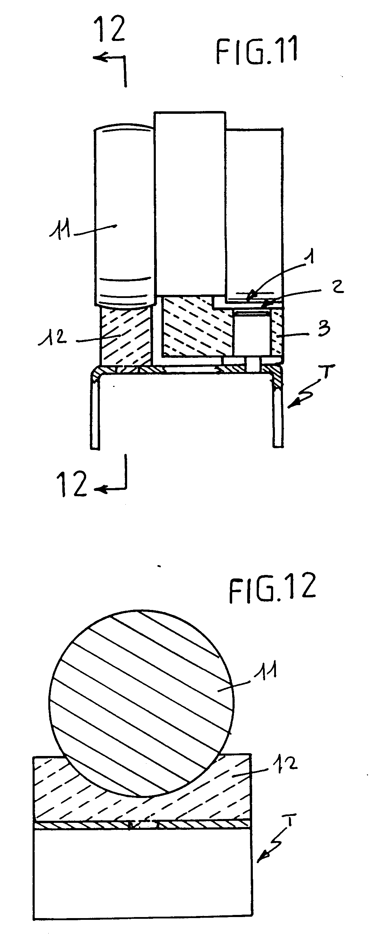

On prévoit également, dans une autre forme de réalisation, comme illustré figures 11 et 12, de réaliser l'articulation, non pas au moyen d'un axe, mais directement par des formes complémentaires de glissement et/ou de pivotement (11) et (12).

Le segment courbe (1b) constitue une portée semi-circulaire de glissement, notamment lors de la flexion des deux éléments fémoral et tibial, comme il sera indiqué dans la suite de la description. D'une manière coaxiale à cette portée de glissement, l'élément fémorale présente une autre portée semi-circulaire (1f) de diamètre supérieur et apte à coopérer à glissement, avec une empreinte en creux complémentaire de l'organe mobile (3) que présente l'élément tibial. La différence de diamètre entre les portées (1b) et (1f) constitue un chemin de guidage entre l'élément fémoral (F) et l'élément tibial (T), notamment au moment de la flexion et de la rotation combinées, comme il sera indiqué dans la suite de la description.

Dans le cas d'une exo-prothèse, les éléments tibial et fémoral sont agencés pour être accouplés au moignon de la cuisse par exemple, et à un ensemble constituant le corps de la jambe avec le pied (figure 10). De telles dispositions ne sont pas décrites en détail car elles peuvent présenter de nombreuses formes de réalisation et ne font pas partie de l'objet spécifique de l'invention.

De même, toujours dans le cas d'une exo-prothèse, on prévoit d'asservir les éléments fémoral et tibial à tout dispositif de mise en tension susceptible de remplacer l'appareil ligamentaire. Par exemple, comme le montre la figure 9, l'élément fémoral peut être accouplé par une courroie (8) à une poulie (9) en étant assujetti à un organe élastique (10) et à un élément amortisseur. La poulie (9) peut faire office d'amortisseur.

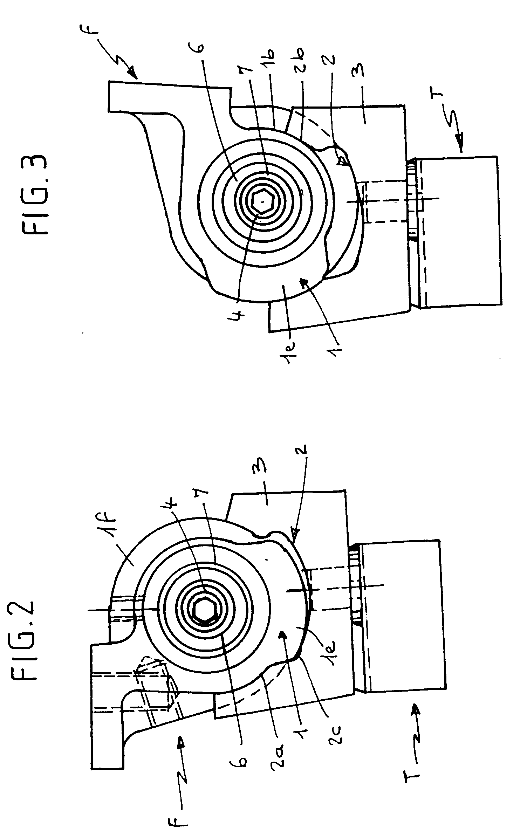

Il convient d'analyser le fonctionnement de l'ensemble prothétique selon l'invention, en se référant notamment aux figures 2, 3, 4 et 5.

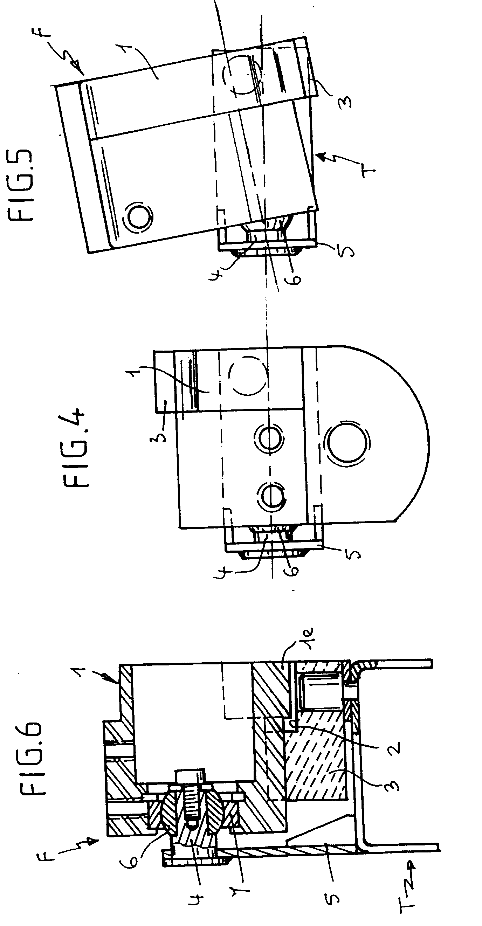

En position extension (figures 2 et 4) les segments courbes (1a) et (2a) des parties complémentaires mâle (1) et femelle (2) sont en contact. Il en est de même en ce qui concerne une partie du segment courbe (1e) qui est en contact avec le segment courbe (2e) de la forme femelle (2). Le segment rectiligne (1d) est en position de butée contre le profil de raccordement (2h) entre les segments (2b) et (2d).

Au début de la flexion, le segment rectiligne (1c) et la partie de segment courbe (1e) échappent respectivement, les segments (2c) et (2e), l'autre partie opposée de segment courbe (1e) venant en contact au niveau du segment rectiligne (2g), le segment courbe (1b) étant en appui contre le segment (2b) (figure 3). Il en résulte, d'une manière concomitante, le pivotement angulaire de l'élément tibial (T), compte tenu du pivotement angulaire de la partie (3) de l'élément tibial présentant l'empreinte en creux (2).

Au fur et à mesure de la flexion, le segment courbe (1e) et la portée (1b) glissent sur les segments courbe (2a) et (2b), jusqu'en position de butée. Il apparait donc, au début de la flexion, un mouvement volontaire de rotation interne de l'élément tibial (T) par rapport à l'élément fémoral (F), selon un angle limité déterminé par les distances (d) et (d1) des parties femelle et mâle comme indiqué précédemment. L'axe (4) et la rotule (6) permettent la combinaison du mouvement de flexion et de rotation de l'élément tibial. A noter que dans ce mouvement de flexion, quel que soit le degré considéré, on a toujours trois points d'appui. En outre, les formes déterminées des parties complémentaires mâle (1) et femelle (2) permettent d'obtenir un roulage et un glissement au fur et mesure de la flexion.

Dans le cas d'une application de l'ensemble prothétique selon l'invention à une prothèse interne, l'axe transversal d'articulation est supprimé.

Les avantages ressortent bien de la description, en particulier on souligne et on rappelle la rotation interne limitée de l'élément tibial par rapport à l'élément fémoral, au début de la flexion et d'une manière automatique, correspondant au mouvement naturel d'articulation du genou, évitant ainsi de solliciter notamment l'articulation de la hanche

## Revendications

1. Ensemble prothétique pour prothèse du genou, comprenant un élément fémoral (F) et un élément tibial (T) présentant des formes complémentaires d'articulation mâle (1)-femelle (2) d'appui et de glissement, caractérisé :
- et en ce que la forme femelle (2) est constituée par deux segments courbes concentriques (2a) (2b) et de rayons différents (r) (r1), lesdits segments (2a) (2b) étant reliés par approximativement un quart de cercle (2c) (2d) respectivement à deux autres segments courbes (2e) (2f) dont les rayons (r2) sont identiques mais dont les centres (O) (O1) sont alignés et décalés d'une distance (d), l'un de ces centres (O1) correspondant au centre des segments courbes concentriques (2a) (2b), les segments courbes de raccordement (2e) (2f) étant reliés par un segment rectiligne (2g) correspondant à ladite distance (d),
- en ce que la forme mâle (1) est constituée par deux segments courbes (1a) (1b) de rayons différents (r3) (r4) correspondant aux rayons (r) (r1) des segments courbes concentriques (2a) (2b) de la forme femelle (2), et dont les centres (O2) (O3) sont décalés d'une valeur (d1) correspondant au décalage (d) des centres (O) (O1) des segments courbes concentriques (2a) (2b) de la forme femelle (2), lesdits segments courbes (1a) (1b) de la forme mâle (1) étant reliés par deux segments rectilignes (1c) (1d) à un seul segment courbe (1e) dont le rayon (r3) correspond aux rayons (r2) de chaque segment courbe de raccordement (2e) (2f) de la forme femelle (2), les segments courbes (1b) concentriques et rectilignes (1d) de la forme mâle du côté postérieur sont réunis par un rayon (1h), tandis que les segments courbes concentriques (2b) et le quart de cercle (2d) de la forme femelle du côté postérieur sont réunis par un rayon (2h), de longueur égale ou inférieure à la longueur du rayon (1h) de la forme mâle (1),
lesdites formes (1) et (2) présentant chacune des profils en section sagittale aptes à provoquer automatiquement, en combinaison avec un mouvement de flexion correspondant au début de la flexion du genou, un mouvement de rotation interne de l'élément tibial (T) par rapport à l'élément fémoral (F) selon un angle limité déterminé.

2. Ensemble selon la revendication 1, caractérisé en ce que les formes complémentaires (1) et (2) sont établies d'un des deux côtés de chaque élément (F) (T) et notamment du côté latéral interne.

3. Ensemble selon la revendication 2, caractérisé en ce qu'à l'opposé des formes complémentaires (1) et (2), chaque élément présente des agencements d'articulation.

4. Ensemble selon la revendication 3, caractérisé en ce que les agencements d'articulation sont constitués par des formes d'appui complémentaires de pivotement et/ou de glissement.

5. Ensemble selon la revendication 3, caractérisé en ce que les agencements d'articulation sont constitués par un axe transversal (4).

6. Ensemble selon la revendication 5, caractérisé en ce que l'axe transversal (4) est accouplé à une partie fixe (5) de l'élément tibial (T) et présente, à son extrémité libre, une rotule (6) engagée dans une forme complémentaire (7) de l'élément fémoral (F).

7. Ensemble selon la revendication 1, caractérisé en ce que la forme mâle (1) est établie au niveau de l'élément fémoral (F), tandis que la forme femelle (2) est établie au niveau de l'élément tibial (T).

8. Ensemble selon la revendication 1, caractérisé en ce que la forme femelle (2) est établie dans un organe rapporté (3) solidaire, avec capacité de pivotement angulaire de l'élément tibial (T).

9. Ensemble selon la revendication 1, caractérisé en ce que les éléments fémoral (F) et tibial (T) sont asservis à un dispositif de mise en tension et d'amortissement (8) (9) (10).

## Claims

1. Prosthesis assembly for knee prosthesis comprising a femoral element (F) and a tibial element (T) having matching articulated male (1) and female (2) support and sliding surfaces, characterised:
- in that the female shape (2) consists of two curved concentric segments (2a) (2b) of different radii (r) (r1), said segments (2a) (2b) being joined by approximately a quarter circle (2c) (2d) respectively to two other curved segments (2e) (2f) the radii (r2) of which are identical but of which the centres (O) (O1) are aligned and offset by a distance (d), one of these centres (O1) corresponding to the centre of the curved concentric segments (2a) (2b), the curved connecting segments (2e) (2f) being joined by a straight segment (2g) corresponding to said distance (d),
- and in that the male shape (1) consists of two curved segments (1a) (1b) of different radii (r3) (r4) corresponding to the radii (r) (r1) of curved concentric segments (2a) (2b) of the female shape (2) and of which the centres (O2) (O3) are offset by a value (d1) corresponding to the offset (d) of centres (O) (O1) of curved concentric segments (2a) (2b) of the female shape (2), said curved segments (1a) (1b) of the male shape (1) being linked by two straight segments (1c) (1d) to a single curved segment (1e) the radius of which (r3) corresponds to the radii (r2) of each curved connecting segment (2e) (2f) of female shape (2), the curved concentric (1b) and straight segments (1d) of the male shape on the posterior side are joined by a radius (1h) whereas the curved concentric segments (2b) and the quarter circle (2d) of the female shape on the posterior side are joined by a radius (2h) of length equal to or less than the length of radius (1h) of the male shape (1),
said shapes (1) and (2) each having sagittal cross-sectional profiles capable of automatically causing, in conjunction with a flexing movement corresponding to the start of flexing of the knee, an internal movement of rotation of tibial element (T) relative to femoral element (F) over a predetermined limited angle.

2. Assembly as claimed in claim 1, characterised in that the matching shapes (1) and (2) are located on one of the two sides of each element (F) (T) and, in particular, on the internal lateral side.

3. Assembly as claimed in claim 2, characterised in that, opposite matching shapes (1) and (2), each element has articulation features.

4. Assembly as claimed in claim 3, characterised in that the articulation features consist of matching support shapes that allow pivoting and/or sliding.

5. Assembly as claimed in claim 3, characterised in that the articulation features consist of a transverse pin (4).

6. Assembly as claimed in claim 5, characterised in that the transverse pin (4) is joined to a fixed part (5) of tibial element (T) and its free end has a ball joint (6) engaged in a matching shape (7) of femoral element (F).

7. Assembly as claimed in claim 1, characterised in that the male shape (1) is located at the level of femoral element (F) whereas the female shape (2) is located at the level of the tibial element (T).

8. Assembly as claimed in claim 1, characterised in that the female shape (2) is located in a separately mounted device (3) which is physically connected to and capable of pivoting angularly relative to the tibial element (T).

9. Assembly as claimed in claim 1, characterised in that the femoral (F) and tibial (T) elements are controlled by a tensioning and damping device (8) (9) (10).

## Patentansprüche

1. Knieprothesensystem mit einem femoralen Element (F) und einem tibialen Element (T), die komplementäre Gelenkformen - Gelenkkopf (1) und Gelenkpfanne (2) - für Auflage und Gleitbewegung aufweisen, dadurch gekennzeichnet, daß:
- die Gelenkpfannenform (2) aus zwei gekrümmten konzentrischen Segmenten (2a) (2b) mit unterschiedlichen Radien (r) (r1) besteht, wobei die Segmente (2a) (2b) durch ungefähr einen Viertelkreis (2c) (2d) jeweils mit zwei anderen gekrümmten Segmenten (2e) (2f) verbunden sind, deren Radien (r2) identisch sind, aber deren Mittelpunkte (O) (O1) in einer Linie liegen und um einen Abstand (d) versetzt sind, wobei einer der Mittelpunkte (O1) dem Mittelpunkt der gekrümmten konzentrischen Segmente (2a) (2b) entspricht, während die gekrümmten Anschlußsegmente (2e) (2f) durch ein geradliniges Segment (2g) verbunden sind, das dem besagten Abstand (d) entspricht;
- die Gelenkkopfform (1) aus zwei gekrümmten Segmenten (1a) (1b) mit unterschiedlichen Radien (r3) (r4) besteht, die den Radien (r) (r1) der gekrümmten konzentrischen Segmente (2a) (2b) der Gelenkpfannenform (2) entsprechen und deren Mittelpunkte (O2) (O3) um einen Wert (d1) versetzt sind, der dem Abstand (d) der Mittelpunkte (O) (O1) der gekrümmten konzentrischen Segmente (2a) (2b) der Gelenkpfannenform (2) entspricht, wobei die gekrümmten Segmente (1a) (1b) der Gelenkkopfform (1) durch zwei geradlinige Segmente (1c) (1d) mit einem einzigen gekrümmten Segment (1e) verbunden sind, dessen Radius (r3) den Radien (r2) jedes gekrümmten Anschlußsegments (2e) (2f) der Gelenkpfannenform (2) entspricht; die gekrümmten (1b) konzentrischen und geradlinigen (1d) Segmente der Gelenkkopfform auf der hinteren Seite sind durch einen Radius (1h) verbunden, während die gekrümmten konzentrischen Segmente (2b) und der Viertelkreis (2d) der Gelenkpfannenform auf der hinteren Seite durch einen Radius (2h) verbunden sind, dessen Länge gleich oder geringer ist als die Länge des Radius (1h) der Gelenkkopfform (1), wobei die Formen (1) und (2) jeweils im sagittalen Querschnitt Profile aufweisen, die geeignet sind, in Verbindung mit einer dem Beginn der Kniebeugung entsprechenden Beugebewegung automatisch in einem bestimmten begrenzten Winkel eine Innenrotationsbewegung des tibialen Elements (T) gegenüber dem femoralen Element (F) zu bewirken.

2. System nach Anspruch 1, dadurch gekennzeichnet, daß die sich ergänzenden Formen (1) und (2) auf einer der beiden Seiten jedes Elements (F) (T) und insbesondere auf der inneren seitlichen Seite ausgebildet sind.

3. System nach Anspruch 2, dadurch gekennzeichnet, daß jedes Element auf der Gegenseite der sich ergänzenden Formen (1) und (2) Gelenkvorkehrungen aufweist.

4. System nach Anspruch 3, dadurch gekennzeichnet, daß die Gelenkvorkehrungen aus komplementären Stützformen für Dreh- und/oder Gleitbewegungen bestehen.

5. System nach Anspruch 3, dadurch gekennzeichnet, daß die Gelenkvorkehrungen aus einer quer verlaufenden Achse (4) bestehen.

6. System nach Anspruch 5, dadurch gekennzeichnet, daß die Querachse (4) mit einem feststehenden Teil (5) des tibialen Elements (T) verbunden ist und an ihrem freien Ende eine Kniescheibe (6) aufweist, die in eine ergänzende Form (7) des femoralen Elements (F) eingeführt ist.

7. System nach Anspruch 1, dadurch gekennzeichnet, daß die Gelenkkopfform (1) im Bereich des femoralen Elements (F) ausgebildet ist, während die Gelenkpfannenform (2) im Bereich des tibialen Elements (T) ausgebildet ist.

8. System nach Anspruch 1, dadurch gekennzeichnet, daß die Gelenkpfannenform (2) in einem angesetzten, mit dem tibialen Element (T) fest verbundenen und winkelig schwenkbaren Organ (3) ausgebildet ist.

9. System nach Anspruch 1, dadurch gekennzeichnet, daß das femorale (F) und das tibiale Element (T) von einer Spannungs- und Dämpfungsvorrichtung (8) (9) (10) abhängig sind.
